(19) Europäisches Patentamt
European Patent Office
Office européen des brevets

(11) EP 2 271 684 B1

(12) **FASCICULE DE BREVET EUROPEEN**

(45) Date de publication et mention de la délivrance du brevet:
**05.06.2019 Bulletin 2019/23**

(21) Numéro de dépôt: **09742230.7**

(22) Date de dépôt: **02.04.2009**

(51) Int Cl.:
***C08G 18/02*** (2006.01) ***C08G 18/09*** (2006.01)
***C08G 18/18*** (2006.01) ***C07D 251/34*** (2006.01)

(86) Numéro de dépôt international:
**PCT/FR2009/050562**

(87) Numéro de publication internationale:
**WO 2009/136032 (12.11.2009 Gazette 2009/46)**

(54) **PROCÉDÉ D'OLIGOMÉRISATION DES ISOCYANATES EN CONTINU**

VERFAHREN ZUR KONTINUIERLICHEN OLIGOMERISIERUNG VON ISOCYANATEN

PROCESS FOR CONTINUOUS OLIGOMERIZATION OF ISOCYANATES

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **03.04.2008 FR 0852233**

(43) Date de publication de la demande:
**12.01.2011 Bulletin 2011/02**

(73) Titulaires:
- **Centre National de la Recherche Scientifique (C.N.R.S.)**
  **75016 Paris (FR)**
- **Institute National Polytechnique de Toulouse**
  **31029 Toulouse Cedex 4 (FR)**
- **Vencorex France**
  **69800 Saint-Priest (FR)**

(72) Inventeurs:
- **KABIR, Hocine**
  **F-69360 Serezin du Rhone (FR)**
- **SCHWARZ, Johannes**
  **F-69009 Lyon (FR)**
- **GOURDON, Christophe**
  **F-31500 Toulouse (FR)**
- **ELGUE, Sébastien**
  **F-31450 Baziege (FR)**

(74) Mandataire: **Dennemeyer & Associates S.A.**
**Landaubogen 1-3**
**81373 München (DE)**

(56) Documents cités:
**WO-A-2006/063745 FR-A- 2 804 112**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 271 684 B1

## Description

La présente invention a pour objet un procédé d'oligomérisation d'isocyanates en continu.

De nombreux procédés de préparation de polyisocyanates sont connus et comprennent l'utilisation de divers catalyseurs.

Ainsi, la demande de brevet EP 0 003 765 décrit la cyclotrimérisation partielle de l'isophorone diisocyanate (IPDI ou 1-isocyanato-3,3,5-triméthyl-5-isocyanatométhyl-cyclohexane), via la mise en oeuvre d'un procédé discontinu (ou procédé batch) utilisant en tant que catalyseur un hydroxyde d'un hydroxyalkylammonium quaternaire.

La demande de brevet DE 10 2004 060 131 décrit un procédé de préparation de polyisocyanates en continu comprenant l'utilisation d'un catalyseur notamment choisi parmi les sels d'ammoniums quaternaires. Il est précisé que la température dans le réacteur varie de 40°C à 110°C, mais cette température n'est pas uniforme à l'intérieur du réacteur en raison de l'existence de différentes zones de température dans ledit réacteur (zones de préchauffage, de réaction et de refroidissement). Enfin, ce document mentionne uniquement l'utilisation d'un réacteur tubulaire.

La demande de brevet EP 0 927 731 décrit un procédé de préparation en continu d'un polyisocyanate à partir d'IPDI en présence d'hydroxyde ou de carboxylate d'ammonium quaternaire, et à une température de 40 à 120°C, mais cette température n'est pas uniforme à l'intérieur du réacteur en raison de l'existence de différentes zones de température dans ledit réacteur (zones de préchauffage, de réaction et de refroidissement). Enfin, ce document concerne l'utilisation d'un réacteur tubulaire.

D'une manière générale, les catalyseurs notamment à base d'un hydroxyde d'ammonium quaternaire tel que la choline ont une réactivité très forte, ce qui rend difficile le contrôle de la réaction de trimérisation, en particulier lorsque l'isocyanate de départ est un isocyanate aliphatique.

Ainsi, l'utilisation de catalyseurs très actifs de ce type nécessite un contrôle de la température à l'intérieur du réacteur.

La présente invention est définie par les revendications et a pour but de fournir un nouveau procédé de préparation de polyisocyanates en utilisant comme catalyseurs des catalyseurs de très forte réactivité, notamment des hydroxydes d'ammonium quaternaire comme la choline, facile à mettre en oeuvre malgré la réactivité très forte des catalyseurs utilisés.

La présente invention a également pour but de fournir un tel procédé au cours duquel la température à l'intérieur du réacteur utilisé est contrôlée.

La présente invention a également pour but de fournir un nouveau procédé de préparation de polyisocyanates en utilisant comme catalyseurs des catalyseurs de très forte réactivité, notamment des hydroxydes d'ammonium quaternaire comme la choline, dans un réacteur permettant l'évacuation de la chaleur de la réaction.

La présente invention a également pour but de fournir un tel procédé permettant l'ajout simultané du catalyseur et du monomère isocyanate.

La présente invention concerne un procédé d'oligomérisation d'isocyanates comprenant une étape de transformation en continu d'un monomère isocyanate en polyisocyanate contenant des groupes carbamates et/ou allophanates en présence d'un catalyseur, dans lequel l'étape de transformation est effectuée dans un réacteur type réacteur-échangeur à plaques dont le coefficient spécifique d'échange thermique est supérieur ou égal à 200 kW/$m^3$/°C, de préférence de 500 kW/$m^3$/°C à 4000 kW/$m^3$/°C, la température étant constante et maîtrisée à l'intérieur du réacteur tout au long de la réaction, et maintenue entre 65°C et 180°C.

Le procédé de l'invention est donc caractérisé par son caractère continu et par un maintien de la température dans le réacteur dans une gamme donnée. Le procédé de l'invention permet ainsi de maintenir tout au long de la réaction une température constante et maîtrisée à l'intérieur du réacteur par un transfert efficace et maîtrisé des calories.

De façon avantageuse, le réacteur utilisé dans le cadre de la présente invention présente un coefficient spécifique d'échange thermique d'environ 200 kW/$m^3$/°C à environ 14 000 kW/$m^3$/°C.

Le procédé selon l'invention est caractérisé en ce que l'étape de transformation susmentionnée est effectuée en continu dans un réacteur maintenu à une température de 65°C à 180°C, notamment d'environ 65°C à environ 130°C, de préférence d'env iron 80°C à environ 100°C, et plus particulièrement à une température d'environ 90°C à environ 95°C.

Ainsi, la mise en oeuvre d'un procédé en continu permet un meilleur contrôle de la réaction par rapport à la mise en oeuvre d'un procédé discontinu (procédé batch), ainsi qu'un contrôle du taux de transformation.

Cette mise en oeuvre du procédé en continu permet également de réduire le volume de l'équipement utilisé et d'obtenir un gain de productivité. Par ailleurs, le procédé en continu augmente la sécurité de conduite.

Les réacteurs utilisés dans le cadre de la présente invention présentent un volume plus faible par rapport aux réacteurs utilisés dans les procédés batch.

Ainsi, en cas de dérive du procédé, le volume réactionnel engagé étant beaucoup plus faible, la gestion du procédé en termes de sécurité est plus aisée.

Le procédé de la présente invention permet de moduler et de contrôler le taux de transformation. Le contrôle du taux de transformation, et plus particulièrement son maintien, permet de contrôler les propriétés physico-chimiques

du produit obtenu (trimère), telles que la viscosité ou le taux de fonctions NCO.

Le terme oligomérisation désigne un processus de transformation d'un monomère, ou d'un mélange de monomères, en un oligomère, qui est une substance composée de molécules de masse moléculaire intermédiaire essentiellement constituées par la répétition en petit nombre d'atomes ou de groupes d'atomes, appelés « unités constitutives » et dérivant de molécules de faible masse moléculaire.

Le coefficient spécifique d'échange thermique est désigné par US/V, où U représente le coefficient d'échange thermique, S représente la surface du réacteur et V représente le volume du réacteur.

Le coefficient U est lié à la conductivité thermique du matériau du réacteur et donc à la nature du matériau du réacteur, ainsi qu'au type de procédé mis en oeuvre, c'est-à-dire par exemple en fonction de l'agitation et de la turbulence des fluides ou de la nature des fluides utilisés.

Le coefficient d'échange thermique U entre deux fluides 'a' et 'b' de part et d'autre d'une plaque d'épaisseur 'e' est défini comme la somme des résistances au transfert thermique tel que : $1/U = 1/h_a + e/\lambda + 1/h_b$, $h_a$ et $h_b$ représentant les résistances de convection de chacun des fluides et $\lambda$ la conductivité thermique du matériau les séparant.

En désignant $\Delta T_a = (T_a)^e - (T_a)^s$ et $\Delta T_b = (T_b)^e - (T_b)^s$ respectivement la variation de température entre l'entrée et la sortie du fluide chaud et du fluide froid, on définit la température logarithmique moyenne par :

$$\Delta T_m l = (\Delta T_a - \Delta T_b) / Ln[((T_a)^e - (T_b)^e) / ((T_a)^s - (T_b)^s)]$$

L'énergie, ou le flux thermique, ($\Phi$), transférée du fluide chaud 'a' au fluide froid 'b' est alors exprimée par : $\Phi = US\ \Delta T_m l$

Cette énergie transférée correspond :

- à l'énergie fournie par le fluide chaud et qui se refroidit de $\Delta T_a$ :

$$W = Q_a\ Cp_a\ \Delta T_a$$

- à l'énergie reçue par le fluide froid et qui s'échauffe de $\Delta T_b$ :

$$W = - Q_b\ Cp_b\ \Delta T_b,$$

Q et Cp étant respectivement le débit et la capacité calorifique des fluides traversant le procédé.

Le bilan de conservation de l'énergie conduit aux relations de conservation suivantes

$$\Phi = US\ \Delta T_m l = Q_a\ Cp_a\ \Delta T_a = - Q_b\ Cp_b\ \Delta T_b$$

Les mesures des températures des deux fluides à l'entrée et à la sortie du procédé et la surface d'échange S permettent donc de mesurer le coefficient d'échange thermique U, caractéristique du système.

Ainsi, le coefficient de transfert thermique est calculé comme indiqué ci-dessous, dans un cas où le fluide froid (ou fluide 'utilité') et le fluide chaud que l'on veut refroidir (ou fluide 'procédé') sont tous les deux de l'eau.

L'expérience est menée sur deux plaques : Une plaque 'utilité' qui reçoit le fluide froid et une plaque 'procédé' qui reçoit le fluide chaud. Les deux plaques sont jointes de façon étanche pour permettre le transfert de chaleur du fluide chaud vers le fluide froid. Le fluide 'utilité' est envoyé dans la plaque 'utilité' à un débit $Q_b$ = 0,02867 kg/s et à une température d'entrée de $(T_b)^e$ = 9,8°C. Le fluide 'procédé' est envoyé dans la plaque 'procédé' à un débit $Q_a$ = 0,00213 kg/s et à une température d'entrée $(T_a)^e$ = 69,6°C. La température du fluide 'utilité' en sortie de la plaque 'utilité' est mesurée à $(T_b)^s$ = 11,5°C. La température du fluide 'procédé' en sortie de la plaque 'procédé' est mesurée à $(T_a)^s$ = 46°C. La variation de température du fluide 'utilité' est donc $\Delta T_b$ = -1,7°C. La variation de température du fluide 'procédé' est donc $\Delta T_a$ = 23,6 °C. La capacité calorifique moyenne du fluide 'utilité' entre l'entrée et la sortie de la plaque 'utilité' est égale à $Cp_b$ = 4201 J/kg/°C. La capacité calorifique moyenne du fluide 'procédé' entre l'entrée et la sortie de la plaque 'procédé' est égale à $Cp_a$ = 4179 J/kg/°C. L'énergie reçue par le fluide 'utilité' est donc : $W = -0,02867 \times 4201 \times (-1,7)$ = 205 watt. L'énergie perdue par le fluide 'procédé' est donc : $W = 0,00213 \times 4179 \times 23,6$ = 210 watt. On attribue la différence entre les deux énergies calculées à la précision des sondes de température et des mesures de débit. Dans cet exemple, on prendra comme valeur de l'énergie échangée la moyenne des deux calculées ci-dessus, soit W = (210 + 205)/2 = 207,5 watt.

La température logarithmique moyenne est calculée par la relation indiquée ci-dessus et donne numériquement :

$$\Delta T_m l = [23,6 - (-1,7)] / Ln[(69,6-9,8) / (46-11,5)] = 46°C$$

On en déduit donc : US = 207,5 / 46 = 4,51 W/°C

Connaissant la surface d'échange 'S', on en déduit le coefficient d'échange thermique d'échange 'U' exprimé en $W/m^2/°C$.

Dans le cadre du procédé de l'invention, il est essentiel de maîtriser la température afin de limiter la variation du taux de transformation.

Les réactions mises en oeuvre dans le cadre de la présente invention sont généralement exothermiques.

Parmi les catalyseurs utilisés dans le procédé de l'invention, on peut notamment citer les catalyseurs tels que ceux décrits dans la demande internationale WO 02/53614 ou WO 01/49766.

Selon un mode de réalisation préféré, le procédé tel que défini ci-dessus comprend l'utilisation d'un catalyseur choisi parmi les hydroxydes d'ammonium quaternaire de formule (I) suivante :

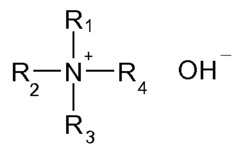

(I)

dans laquelle :

- $R_1$ représente un groupe alkyle, saturé ou non, comprenant de 1 à 20 atomes de carbone, un groupe aryle comprenant de 6 à 12 atomes de carbone, un groupe cycloalkyle comprenant de 5 à 12 atomes de carbone ou un système hétérocyclique à 5 ou 6 maillons comportant un atome d'azote, un atome d'oxygène ou un atome de soufre,
- $R_2$, $R_3$ et $R_4$ représentent, indépendamment l'un de l'autre, un groupe alkyle, saturé ou non, comprenant de 1 à 20 atomes de carbone, un groupe aryle comprenant de 6 à 12 atomes de carbone, lesdits groupes $R_2$, $R_3$ et $R_4$ pouvant être le cas échéant substitués par au moins un groupe hydroxyle ou un groupe thiol.

La notion de maîtrise thermique est essentielle dans le cadre de l'utilisation de ces hydroxydes d'ammonium quaternaire, qui sont sensibles à la température, et ce afin d'éviter la dégradation de ces catalyseurs.

Le contrôle de la température pour le procédé de l'invention est essentiel afin de pouvoir maîtriser l'avancement de la réaction.

Si la température dans le réacteur est inférieure à 65°C, la réaction n'avance pas assez vite.

Lorsque la température dans le réacteur est supérieure à 180°C, la réaction devient trop rapide et donc non contrôlable. Il n'est alors plus possible de contrôler le taux de transformation lié à l'avancement de la réaction.

La présente invention permet de trouver un compromis entre la vitesse de réaction, qui doit être rapide (de 1 à 3 minutes), et la dégradation du catalyseur. Le procédé de l'invention est caractérisé en ce que l'étape de transformation est effectuée dans un réacteur type réacteur-échangeur à plaques.

Pour les réacteurs à plaques, on peut citer la technologie d'Alpha Laval telle que décrite dans les demandes de brevet FR 2 823 995, US 2004/0109798, FR 2 880 967, WO 02/85511, US 2006/0159600, EP 1 562 699, et celle de Chart et BRH Group avec la technologie Marbond™ Hex Reactor (telle que décrite dans les demandes WO 02/37047 et WO 02/58840).

La présente invention concerne également un procédé tel que défini ci-dessus dans lequel l'étape de transformation est effectuée pendant un temps de séjour dans le réacteur inférieur à 1 heure, de préférence inférieur à 30 minutes, et encore de préférence inférieur à 10 minutes, et préférentiellement pendant environ 3 minutes.

Ce temps de séjour réduit est important afin d'adapter un procédé de type batch en un procédé continu, et ce en raison du grand nombre de calories à évacuer. Si le temps de séjour devient trop important, on perd alors l'avantage d'effectuer le procédé en continu.

Les catalyseurs utilisés ici doivent être adéquats pour avoir un temps de séjour limité.

En terme de productivité, l'augmentation est par exemple environ d'un facteur 20 par rapport aux procédés discontinus.

Selon un mode de réalisation avantageux, le procédé de l'invention est caractérisé en ce que le monomère isocyanate et le catalyseur sont ajoutés simultanément dans le réacteur.

De façon préférée, le procédé de l'invention est caractérisé en ce que le monomère isocyanate est choisi dans le groupe constitué des isocyanates aliphatiques, cycloaliphatiques et aromatiques.

Selon un mode de réalisation particulièrement avantageux du procédé tel que défini ci-dessus, le monomère isocyanate est choisi dans le groupe constitué du 1,6-hexaméthylènediisocyanate (HDI), du 1,12-dodécane diisocyanate, du cyclobutane-1,3-diisocyanate, du cyclohexane-1,3 et/ou 1,4-diisocyanate, du 1-isocyanato-3,3,5-triméthyl-5-isocyanatométhylcyclohexane (isophorone diisocyanate - IPDI), des isocyanatométhyloctylènedi-isocyanate (TTI), du dicyclohexyl-méthan-4,4'-diisocyanate, du toluène diisocyanate (TDI), du méthylène bis-phénylisocyanate (MDI), du 2,2,4-triméthyl-1,6-hexaméthylènediisocyanate, du 2,4,4-triméthyl-1,6-hexaméthylènediisocyanate (TMDI), du 2,4- et/ou 2,6-hexahydrotoluylène di-isocyanate ($H_6$TDI), du 2,4- et/ou le 2,6-toluylène diisocyanate, du hexahydro-1,3 et/ou 1,4-phénylène diisocyanate, du 2-méthylpentaméthylène diisocyanate (MPDI), du 1,3- et/ou du 1,4-phénylène diisocyanate, des tétraméthylxylilène diisocyanates (TMXDI), du triphénylméthane-4,4',4"-triisocyanate, du lysine diisocyanate ainsi que des esters de la lysine di- ou tri-isocyanate (LDI ou LTI), du diphénylméthane-2,4' et/ou 4,4'-diisocyanate (MDI), du perhydro 2,4' et/ou 4,4'-diphénylméthane diisocyanate ($H_{12}$MDI), et en général les précurseurs aromatiques aminés ou les carbamates perhydrogénés, des bis-isocyanatométhylcyclohexanes (BIC), notamment les 1,3-BIC et 1,4-BIC, des bis-isocyanatométhylnorbornanes (NBDI) et des oligomères du MDI ou du TDI.

La présente invention concerne également un procédé tel que défini ci-dessus dans lequel le catalyseur est choisi dans le groupe constitué de l'hydroxyde de tétrabutylammonium (TBAOH) et de l'hydroxyde de N,N,N-triméthyl-N-(2-hydroxyéthyl)ammonium (hydroxyde de choline).

Selon un mode de réalisation avantageux, le procédé de l'invention est caractérisé en ce que la quantité de catalyseur utilisée est d'environ 0,01% à environ 0,5 % en poids par rapport au poids de monomère.

La présente invention concerne également un procédé tel que défini ci-dessus pour la préparation de trimère d'hexaméthylène diisocyanate (HDT), comprenant une étape de transformation du 1,6-hexaméthylènediisocyanate (HDI) en présence de N,N,N-triméthyl-N-(2-hydroxyéthyl)ammonium.

La présente invention concerne également un procédé tel que défini ci-dessus pour la préparation de trimère d'hexaméthylène diisocyanate (HDT) comprenant une étape de transformation du 1,6-hexaméthylènediisocyanate (HDI) en présence d'hydroxyde de tétrabutylammonium.

Selon un autre mode de réalisation avantageux, le procédé de l'invention est un procédé tel que défini ci-dessus dans lequel le monomère isocyanate contient plus de 30 ppm de chlore hydrolysable.

Ces gammes en chlore hydrolysable constituent un avantage supplémentaire du procédé continu par rapport au procédé discontinu.
En effet, dans le cadre d'un procédé batch, une partie du catalyseur est consommée par des espèces du type "chlore hydrolysable". Par "chlore hydrolysable", on entend des molécules pour lesquelles le chlore est lié de manière covalente et dont il peut être libéré sous forme $Cl^-$. L'analyse du taux de chlore hydrolysable est effectuée par action d'un mélange eau/méthanol (2h, reflux) sur le monomère en question et détermination de la concentration en $Cl^-$ par des moyens appropriés. La consommation partielle du catalyseur par les espèces du type "chlore hydrolysable" se traduit par un début de réaction retardé et un rendement plus faible pour un isocyanate contenant une concentration d'environ 120 ppm de Cl (voir exemple 3 ci-après).

Or, le procédé continu permet de gommer cette différence. Il n'est donc pas nécessaire, dans le cadre du procédé selon la présente invention, d'adapter les conditions opératoires en fonction des matières premières utilisées.

## DESCRIPTION DES FIGURES

La **Figure 1** représente les profils de température en régime stationnaire en présence du catalyseur (choline) (courbe en trait plein avec les losanges) et en régime stationnaire sans catalyseur (courbe en trait pointillé avec les ronds). Ces courbes représentent la température (en °C) en fonction de la variable z/L, L correspondant à la longueur totale des cannelures des plaques du réacteur et z/L correspondant à la position des sondes de température le long de cette longueur. Ainsi, la valeur z/L=0 correspond à l'entrée du réacteur et la valeur z/L=1 correspond à la sortie du réacteur.

La **Figure 2** représente le profil de température en régime batch en présence du catalyseur choline. La courbe en trait pointillé représente l'évolution de la température (°C) en fonction du temps (min) et la courbe en trait plein avec les ronds noirs représente le taux de transformation de l'HDI (%) en fonction du temps (min).

La **Figure 3** représente le profil de température en régime batch en présence du catalyseur TBAOH. La courbe en trait pointillé représente l'évolution de la température (°C) en fonction du temps (min) et la courbe en trait plein avec les ronds noirs représente le taux de transformation de l'HDI (%) en fonction du temps (min).

La **Figure 4** représente la superposition des profils de température des essais avec l'HDI de teneur élevée en chlore hydrolysable (courbe avec les ronds blancs) et avec l'HDI de teneur faible en chlore hydrolysable (courbe en trait plein). Cette figure représente la variation de température $\Delta T$ (°C) en fonction du temps (min). "B" indique la fin de l'introduction du catalyseur.

## PARTIE EXPÉRIMENTALE

### Exemple 1 : Essais en continu dans un réacteur à plaques

1- Caractéristique du réacteur :

Le réacteur à plaques utilisé est un réacteur inox à l'échelle laboratoire qui permet de traiter des débits de 200 millilitres par heure à 10 litres par heure. Il est constitué de 3 plaques dont la structure (cannelures d'environ 2 mm de diamètre creusées dans la plaque) a été conçue pour favoriser les turbulences du fluide qui la traverse et ainsi offrir des performances de transfert thermique optimales. Pour ce réacteur, les performances ont été caractérisées par un coefficient d'échange thermique (U) de 5 000 Watt par mètre carré et par degré Celsius. Le coefficient U a été mesuré comme indiqué ci-dessus en utilisant de l'eau comme fluide. Les trois plaques disposées en série présentent un volume global de 37,01 $cm^3$ et une surface d'échange de 0,074 $m^2$. Le coefficient spécifique d'échange thermique (US/V) est donc de 8 020 Kilowatt par mètre cube et par degré Celsius.

Chacune de ces plaques est disposée entre deux autres plaques dans lesquelles circule le fluide caloporteur pour l'évacuation des calories. Plusieurs sondes de température sont disposées le long des plaques "procédé" (plaques recevant le mélange réactionnel), afin de mesurer le profil de température dans le réacteur pendant l'essai.

2- Essais continus de trimérisation de l'HDI par catalyse ammonium quaternaire :

Les essais ont consisté à alimenter le réacteur à plaques par co-addition d'hexaméthylène diisocyanate (HDI, Rhodia) et du catalyseur (choline 45% dans méthanol ou tétrabutylammonium hydroxyde 40% dans méthanol, Aldrich) en solution dans l'éthylhexanol (Aldrich).

Le réacteur à plaques est porté à la température de travail désirée, par circulation du fluide caloporteur dans les plaques correspondantes. Les réactifs sont alors alimentés à co-courant ou à contre-courant du fluide caloporteur (notamment huile silicone chauffée au préalable à 90°C) à travers les plaques procédés adjacentes. Dans le cadre des essais décrits ci-après, les réactifs sont alimentés à co-courant. La conversion de l'HDI en HDT se produit dans la microstructure des plaques et conduit à un dégagement de chaleur correspondant à 18 kcal/mole d'HDI converti. C'est ce dégagement de chaleur qu'il convient d'évacuer efficacement et rapidement pour maîtriser la chimie et optimiser la productivité.

a. Essai continu de trimérisation de l'HDI par catalyse choline

L'évaluation des performances thermiques du réacteur à plaques est réalisée en deux temps. Tout d'abord, il est alimenté par l'HDI pur sans la solution catalytique et le profil thermique le long du réacteur est relevé après stabilisation. Cette première phase permet d'obtenir le profil de température de référence en absence de réaction. La solution catalytique (1,3% en poids de catalyseur dans l'éthylhexanol) est alors co-alimentée et le débit total dans le réacteur, donc le temps de séjour, est ajusté pour obtenir un taux de conversion de l'HDI compris entre 20 et 50%. La réaction s'établit et, après atteinte du régime stationnaire (concentration des produits en sortie de réacteur stabilisée), le même profil est relevé. Les deux profils sont comparés sur la **Figure 1.**

Il apparaît que les deux profils de température sont parfaitement superposables. Ceci indique que la chaleur dégagée lors de la réaction est parfaitement évacuée par le réacteur à plaques. C'est, bien entendu, ce que l'on attend de cette technologie mais cela est particulièrement important dans le cas de la catalyse Choline. En effet, la choline a pour caractéristique de se dégrader thermiquement. Une température minimale est nécessaire pour avoir une vitesse de réaction suffisante pour atteindre le taux de conversion désiré pour un temps de passage fixé. Mais cette température ne doit pas être trop élevée, avec le risque de dégrader rapidement le catalyseur et qu'il devienne inactif avant que la réaction atteigne ce taux de conversion. D'où l'intérêt du réacteur à plaques qui permet d'imposer la bonne température, tout en évacuant parfaitement les calories générées par la réaction.

Le tableau ci-dessous montre effectivement la sensibilité de la réaction avec la température et l'effet cité ci-dessus :

| Catalyseur | Température réacteur (°C) | Débit catalyseur (ml/mn) | Débit HDI (kg/h) | choline/HDI (% mole) | Temps de séjour (min) | Taux de conversion HDI (%) |
|---|---|---|---|---|---|---|
| Choline | 97 | 0,204 | 0,6 | 0,065 | 4,4 | 25% |
| Choline | 93 | 0,204 | 0,67 | 0,06 | 3,95 | 35% |

(suite)

| Catalyseur | Température réacteur (°C) | Débit catalyseur (ml/mn) | Débit HDI (kg/h) | choline/HDI (% mole) | Temps de séjour (min) | Taux de conversion HDI (%) |
|---|---|---|---|---|---|---|
| Choline | 91 | 0,18 | 0,58 | 0,06 | 4,5 | 39% |

On constate donc ici que, lorsque la température augmente, le taux de conversion diminue, et ce en raison de la dégradation trop importante du catalyseur. La maîtrise de la température implique donc une maîtrise du taux de conversion.

b. Essai continu de trimérisation de l'HDI par catalyse hydroxyde de tétrabutylammonium (TBAOH)

Ce même réacteur à plaques est testé pour la trimérisation de l'HDI en utilisant l'hydroxyde de tétrabutylammonium (TBAOH) comme catalyseur. La solution catalytique utilisée dans cet essai contient 1,3% en poids de catalyseur TBAOH dans l'éthylhexanol. Ce catalyseur a les mêmes propriétés catalytiques que la Choline pour la synthèse de l'HDT à partir de l'HDI. Il est par contre thermiquement beaucoup plus stable que la Choline. De ce fait l'activité catalytique de la TBAOH est beaucoup plus grande et la cinétique de trimérisation accrue.

Ce type de catalyse est difficilement maîtrisable en réacteur batch ou semi-batch, avec une réaction qui tend à l'emballement thermique.

Elle est parfaitement maîtrisée dans le réacteur à plaques. Dans le tableau ci-dessous, à 82°C, on apprécie l'impact du temps d e séjour dans le réacteur sur le taux de conversion.

| Catalyseur | Température réacteur (°C) | Débit catalyseur (ml/mn) | Débit HDI (kg/h) | TBAOH/HDI (% mole) | Temps de séjour (min) | Taux de conversion HDI (%) |
|---|---|---|---|---|---|---|
| TBAOH | 82 | 0,218 | 0,540 | 0,03 | 4,9 | 40,9 % |
| TBAOH | 82 | 0,224 | 0,620 | 0,027 | 4,3 | 38,7 % |
| TBAOH | 82 | 0,224 | 0,630 | 0,027 | 4,25 | 35 % |
| TBAOH | 82 | 0,224 | 0,635 | 0,027 | 4,2 | 33 % |

Si le temps de séjour est imposé, le taux de conversion peut également être contrôlé par la charge catalytique. Le tableau ci-dessous, montre la sensibilité de la chimie menée en continu vis-à-vis de la charge catalytique :

| Catalyseur | Température réacteur (°C) | Débit catalyseur (ml/mn) | Débit HDI (kg/h) | TBAOH/HDI (% mole) | Temps de séjour (min) | Taux de conversion HDI (%) |
|---|---|---|---|---|---|---|
| TBAOH | 85 | 0,102 | 0,530 | 0,015 | 5,05 | 3,9 |
| TBAOH | 85 | 0,136 | 0,535 | 0,020 | 5 | 24,7 |
| TBAOH | 85 | 0,170 | 0,540 | 0,025 | 4,9 | 39 |

**Exemple 2 : Essais comparatifs en réacteur batch**

A titre de comparaison, ces deux systèmes réactionnels (choline/TBAOH) ont été testés en réacteur agité en verre double enveloppé. La réaction est menée en conditions batch, les réactifs sont ajoutés dans le réacteur, puis le fluide caloporteur qui traverse la double enveloppe est porté graduellement (rampe de température) de la température ambiante jusqu'à 85°C. La température de la double enveloppe est ensuite maintenue à 85°C jusqu 'à l'arrêt de l'expérience.

1- Caractéristiques du réacteur :

Le réacteur double enveloppé en verre possède un diamètre interne de 0,08 m (8 cm). Le volume de réactifs

ajouté dans ce réacteur représente environ 0,3 l (300 ml) ce qui représente une surface de contact avec la paroi d'environ 0,03 $m^2$. Le coefficient de transfert thermique du verre en contact avec une solution organique est classiquement donné à environ 180 $W.m^{-2}.°C^{-1}$. Le coefficient spécifique d'échange thermique (US/V) du réacteur est donc d'environ 18 Kilowatt par mètre cube et par degré Celsius.

2- Performances en réaction batch :

*Conditions opératoires :*

| Catalyseur | Température Calo porteur | Charge HDI (g) | Charge catalytique (g) | Charge Ethylhexanol (g) | Catalyseur/HDI (% mole) |
|---|---|---|---|---|---|
| Choline | Tamb → 85°C | 316,1 | 0,065 | 2,4372 | 0,03 % |
| TBAOH | Tamb → 85°C | 309,8 | 0,142 | 4,925 | 0,03 % |

Les graphiques de la **Figure 2** et de la **Figure 3** donnent les profils de température du milieu réactionnel et de celui de la conversion de l'HDI.

Deux comportements thermiques différents sont obtenus au vu de ces deux essais.

Essai avec la choline : La réaction s'initie vers 70°C et la réaction s'accélère. La chaleur générée n'est pas évacuée efficacement et s'accumule dans le mélange réactionnel. Cela a pour effet d'élever la température du milieu qui elle-même accélère à nouveau la réaction. On est en présence d'un emballement thermique qui mène la température rapidement à environ 135°C. A cette température, le catalyseur est complètement détruit thermiquement (la conversion s'arrête brutalement), puis le milieu réactionnel refroidit lentement en évacuant progressivement ses calories à travers la double enveloppe.

Essai avec la TBAOH : La réaction s'initie aussi vers 70°C avec le même phénomène qu'avec la choline. L'emballement thermique mène la température rapidement à environ 230°C. A cette température, le catalyseur est toujours actif mais la conversion de l'HDI est totale. Cette conversion totale a pris une à deux minutes, puis le milieu réactionnel refroidit lentement en évacuant progressivement ses calories à travers la double enveloppe. Ici, le milieu réactionnel est trop visqueux.

Ces deux exemples montrent un emballement thermique qui n'a pas pu être maîtrisé par l'équipement choisi (ici le réacteur agité). L'extrapolation de cette technologie restera très délicate voire impossible dans le cas de la catalyse TBAOH.

D'où l'intérêt de disposer d'outils et d'équipements qui permettent un contrôle et une maîtrise parfaite de la thermique. Contrôle et maîtrise qui sont quantifiés par une valeur élevée de leur coefficient spécifique de chaleur qui les caractérise. Les réacteurs à plaques offrent cette propriété.

**Exemple 3 : Essais sur l'impact du chlore hydrolysable**

A titre de comparaison, le système catalytique choline hydroxyde a été testé en réacteur agité en verre double enveloppé avec deux monomères HDI de teneurs en chlore hydrolysable différentes (~25 et 120 ppm exprimés en chlorure). La réaction est menée en conditions batch, le monomère est placé dans le réacteur, puis le fluide caloporteur qui traverse la double enveloppe est porté à 85°C. La température de la double enveloppe est ensuite maintenue à 85°C jusqu'à l'arrêt de l'expérience. La solution catalytique (1,3% en poids de choline hydroxyde dans le 2-éthylhexanol) est introduite progressivement via un pousse seringue dans le milieu réactionnel.

Le réacteur utilisé est identique à celui utilisé dans l'exemple 2. Le volume de réactifs ajouté dans ce réacteur représente environ 0,8 à 1 l (1 000 ml), ce qui représente une surface de contact avec la paroi de 0,04 à 0,05 $m^2$. Le coefficient de transfert thermique du verre en contact avec une solution organique est classiquement donné à environ 180 $W.m^{-2}.°C^{-1}$. Le coefficient spécifique d'échange thermique (US/V) du réacteur est donc d'environ 8 à 9 Kilowatt par mètre cube et par degré Celsius.

Performances en réaction batch :

*Conditions opératoires :*

| HDI - teneur en Cl hydrolysable | Température Calo porteur | Charge HDI (g) | Charge de la solution catalytique à 1,3% en poids de choline hydroxyde (g) | Temps introduction [min] | Catalyseur/HDI (% mole) | Taux de transformation (HDI) |
|---|---|---|---|---|---|---|
| 25 ppm Cl | 85°C | 1 000 g | 8,0 | 60 | 0,014% | 63% |
| 120 ppm Cl | 85°C | 800 g | 6,4 | 60 | 0,014% | 53% |

Essai avec un HDI de teneur faible en chlore hydrolysable : La réaction commence 10 min environ après le début de l'ajout du catalyseur. La chaleur générée dans la première partie n'est pas évacuée complètement ce qui fait monter la température du milieu. Le cryothermostat compense et la température du milieu réactionnel chute avant que l'augmentation de la température ne devienne linéaire, signe de l'accumulation du catalyseur dans le milieu réactionnel et l'avancement de la réaction. Après l'arrêt de la coulée de catalyseur, la réaction s'arrête lentement

Essai avec un HDI de teneur élevée en chlore hydrolysable : La réaction commence avec un décalage de 6 minutes environ par rapport au HDI de faible teneur en chlore. La quantité de chaleur générée lors de cette phase est moins importante et la température augmente progressivement sans à-coups.

La superposition des deux profils de température **(Figure 4)** montre une activité catalytique plus importante pour l'essai « HDI - faible teneur en chlore », dès le début de réaction, ce qui est confirmé par le taux de transformation (TT) supérieur (63% contre 53%), étant entendu que les deux essais (HDI avec une faible teneur en chlore hydrolysable ou HDI avec une teneur élevée en chlore hydrolysable) sont effectués dans des conditions et des installations identiques.

Dans le cadre de la présente invention, l'injection conjointe du monomère HDI et du catalyseur permet de fonctionner avec un ratio optimal pour l'oligomérisation ce qui, associé à un temps de séjour court, permet de gommer l'impact du chlore hydrolysable. L'utilisation d'un réacteur en continu, et plus particulièrement du réacteur décrit ci-dessus, permet donc une plus grande flexibilité en ce qui concerne la qualité des matières premières.

## Revendications

1. Procédé d'oligomérisation d'isocyanates comprenant une étape de transformation en continu d'un monomère isocyanate en polyisocyanate contenant des groupes carbamates et/ou allophanates en présence d'un catalyseur, dans lequel l'étape de transformation est effectuée dans un réacteur type réacteur-échangeur à plaques dont le coefficient spécifique d'échange thermique est supérieur ou égal à 200 $kW/m^3/°C$, la température étant constante et maîtrisée à l'intérieur du réacteur tout au long de la réaction, et maintenue entre 65°C et 180°C .

2. Procédé selon la revendication 1, **caractérisé en ce que** le catalyseur est choisi parmi les hydroxydes d'ammonium quaternaire de formule (I) suivante :

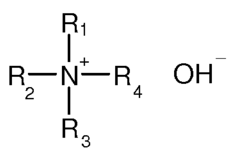

(I)

dans laquelle :

- $R_1$ représente un groupe alkyle, saturé ou non, comprenant de 1 à 20 atomes de carbone, un groupe aryle comprenant de 6 à 12 atomes de carbone, un groupe cycloalkyle comprenant de 5 à 12 atomes de carbone ou un système hétérocyclique à 5 ou 6 maillons comportant un atome d'azote, un atome d'oxygène ou un atome de soufre,
- $R_2$, $R_3$ et $R_4$ représentent, indépendamment l'un de l'autre, un groupe alkyle, saturé ou non, comprenant de 1 à 20 atomes de carbone, un groupe aryle comprenant de 6 à 12 atomes de carbone, lesdits groupes $R_2$, $R_3$ et $R_4$ pouvant être le cas échéant substitués par au moins un groupe hydroxyle ou un groupe thiol.

3. Procédé selon la revendication 1 ou 2, **caractérisé en ce que** l'étape de transformation est effectuée pendant un

temps de séjour dans le réacteur inférieur à 1 heure.

4. Procédé selon l'une quelconque des revendications 1 à 3, **caractérisé en ce que** le monomère isocyanate et le catalyseur sont ajoutés simultanément dans le réacteur.

5. Procédé selon l'une quelconque des revendications 1 à 4, **caractérisé en ce que** le monomère isocyanate est choisi dans le groupe constitué des isocyanates aliphatiques, cycloaliphatiques et aromatiques.

6. Procédé selon l'une quelconque des revendications 1 à 5, **caractérisé en ce que** le monomère isocyanate est choisi dans le groupe constitué du 1,6-hexaméthylènediisocyanate, du 1,12-dodécane diisocyanate, du cyclobutane-1,3-diisocyanate, du cyclohexane-1,3 et/ou 1,4-diisocyanate, du 1-isocyanato-3,3,5-triméthyl-5-isocyanatométhyl-cyclohexane, des isocyanatométhyloctylène-diisocyanates, du dicyclohexyl-méthan-4,4'-diisocyanate, du toluène diisocyanate, du méthylène bis-phénylisocyanate, du 2,2,4-triméthyl-1,6-hexaméthylènediisocyanate, du 2,4- et/ou 2,6-hexahydrotoluylène di-isocyanate, du 2,4- et/ou le 2,6-toluylène diisocyanate, du hexahydro-1,3 et/ou 1,4-phénylène diisocyanate, du 2-méthylpentaméthylène diisocyanate, du 1,3- et/ou du 1,4-phénylène diisocyanate, des tétraméthylxylilène diisocyanates, du triphénylméthane-4,4',4"-triisocyanate, du lysine diisocyanate ainsi que des esters de la lysine di- ou tri-isocyanate, du diphénylméthane-2,4' et/ou 4,4'-diisocyanate, du perhydro 2,4' et/ou 4,4'-diphénylméthane diisocyanate, et en général les précurseurs aromatiques aminés ou les carbamates perhydrogénés, des bis-isocyanatométhylcyclo-hexanes, notamment les 1,3-BIC et 1,4-BIC, des bis-isocyanatométhyl-norbornanes et des oligomères du MDI ou du TDI.

7. Procédé selon l'une quelconque des revendications 1 à 6, **caractérisé en ce que** le catalyseur est choisi dans le groupe constitué de l'hydroxyde de tétrabutylammonium et de l'hydroxyde de N,N,N-triméthyl-N-(2-hydroxyéthyl)ammonium.

8. Procédé selon l'une quelconque des revendications 1 à 7, **caractérisé en ce que** la quantité de catalyseur utilisée est de 0,01% à 0,5% en poids par rapport au poids de monomère.

9. Procédé selon l'une quelconque des revendications 1 à 8, de préparation de trimère d'hexaméthylène diisocyanate comprenant une étape de transformation du 1,6-hexaméthylènediisocyanate en présence de N,N,N-triméthyl-N-(2-hydroxyéthyl)ammonium.

10. Procédé selon l'une quelconque des revendications 1 à 8, de préparation de trimère d'hexaméthylène diisocyanate comprenant une étape de transformation du 1,6-hexaméthylènediisocyanate en présence d'hydroxyde de tétrabutylammonium.

11. Procédé selon l'une quelconque des revendications 1 à 10, dans lequel le monomère isocyanate contient plus de 30 ppm de chlore hydrolysable.

**Patentansprüche**

1. Verfahren zur Oligomerisierung von Isocyanaten, umfassend einen kontinuierlichen Umwandlungsschritt eines Isocyanatmonomers in Polyisocyanat, das Carbamat- und/oder Allophanat-Gruppen enthält, in Gegenwart eines Katalysators, wobei der Umwandlungsschritt in einem Reaktor vom Typ Reaktor-Plattenaustauscher durchgeführt wird, dessen spezifischer Wärmeübergangskoeffizient größer oder gleich 200 kW/m$^3$/°C ist, wobei die Temperatur konstant ist und im Verlauf der Reaktion im Inneren des Reaktors geregelt und zwischen 65 °C und 180 °C gehalten wird.

2. Verfahren nach Anspruch 1, **dadurch gekennzeichnet, dass** der Katalysator aus quartären Ammoniumhydroxiden der folgenden Formel (I) ausgewählt ist:

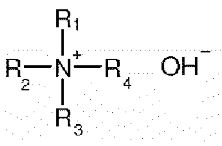
(I)

wobei:

- $R_1$ eine gesättigte oder ungesättigte Alkylgruppe mit 1 bis 20 Kohlenstoffatomen, eine Arylgruppe mit 6 bis 12 Kohlenstoffatomen, eine Cycloalkylgruppe mit 5 bis 12 Kohlenstoffatomen oder ein heterocyclisches System mit 5 oder 6 Gliedern, das ein Stickstoffatom, ein Sauerstoffatom oder ein Schwefelatom enthält, darstellt,
- $R_2$, $R_3$ und $R_4$ unabhängig voneinander eine gesättigte oder ungesättigte Alkylgruppe mit 1 bis 20 Kohlenstoffatomen, eine Arylgruppe mit 6 bis 12 Kohlenstoffatomen darstellt, wobei die Gruppen $R_2$, $R_3$ und $R_4$ gegebenenfalls durch mindestens eine Hydroxylgruppe oder eine Thiolgruppe substituiert sein können.

**3.** Verfahren nach Anspruch 1 oder 2, **dadurch gekennzeichnet, dass** der Umwandlungsschritt während einer Verweilzeit im Reaktor von weniger als 1 Stunde durchgeführt wird.

**4.** Verfahren nach einem der Ansprüche 1 bis 3, **dadurch gekennzeichnet, dass** das Isocyanatmonomer und der Katalysator gleichzeitig in den Reaktor gegeben werden.

**5.** Verfahren nach einem der Ansprüche 1 bis 4, **dadurch gekennzeichnet, dass** das Isocyanatmonomer aus der Gruppe bestehend aus aliphatischen, cycloaliphatischen und aromatischen Isocyanaten ausgewählt ist.

**6.** Verfahren nach einem der Ansprüche 1 bis 5, **dadurch gekennzeichnet, dass** das Isocyanatmonomer aus der Gruppe bestehend aus 1,6-Hexamethylendiisocyanat, 1,12-Dodecandiisocyanat, Cyclobutan-1,3-diisocyanat, Cyclohexan-1,3- und/oder-1,4-diisocyanat, 1-Isocyanato-3,3,5-trimethyl-5-isocyanatomethylcyclohexan, Isocyanatomethyloctylendiisocyanaten, Dicyclohexylmethan-4,4'-diisocyanat, Toluoldiisocyanat, Methylen-bis-phenylisocyanat, 2,2,4-Trimethyl-1,6-hexamethylendiisocyanat, 2,4- und/oder 2,6-Hexahydrotoluylendiisocyanat, 2,4- und/oder 2,6-Toluylendiisocyanat, Hexahydro-1,3- und/oder -1,4-phenylendiisocyanat, 2-Methylpentamethylendiisocyanat, 1,3- und/oder 1,4-Phenylendiisocyanat, Tetramethylxylylendiisocyanaten, Triphenylmethan-4,4',4"-triisocyanat, Lysindiisocyanat sowie Estern von Lysindi- oder -triisocyanat, Diphenylmethan-2,4'-und/oder -4,4'-diisocyanat, Perhydro-2,4'- und/oder -4,4'-diphenylmethandiisocyanat sowie allgemein aus aromatischen aminierten Vorstufen oder perhydrierten Carbamaten, Bisisocyanatomethylcyclohexanen, insbesondere 1,3-BIC und 1,4-BIC, Bisisocyanatomethylnorbornanen sowie Oligomeren von MDI oder TDI ausgewählt ist.

**7.** Verfahren nach einem der Ansprüche 1 bis 6, **dadurch gekennzeichnet dass** der Katalysator aus der Gruppe bestehend aus Tetrabutylammoniumhydroxid und N,N,N-Trimethyl-N-(2-hydroxyethyl)ammoniumhydroxid ausgewählt ist.

**8.** Verfahren nach einem der Ansprüche 1 bis 7, **dadurch gekennzeichnet, dass** die verwendete Katalysatormenge 0,01 Gew.-% bis 0,5 Gew.-% bezogen auf das Monomergewicht beträgt.

**9.** Verfahren nach einem der Ansprüche 1 bis 8 zur Herstellung des Trimers Hexamethylendiisocyanat, umfassend einen Umwandlungsschritt von 1,6-Hexamethylendiisocyanat in Gegenwart von N,N,N-Trimethyl-N-(2-hydroxyethyl)ammonium .

**10.** Verfahren nach einem der Ansprüche 1 bis 8 zur Herstellung des Trimers Hexamethylendiisocyanat, umfassend einen Umwandlungsschritt von 1,6-Hexamethylendiisocyanat in Gegenwart von Tetrabutylammoniumhydroxid .

**11.** Verfahren nach einem der Ansprüche 1 bis 10, wobei das Isocyanatmonomer mehr als 30 ppm hydrolysierbares Chlor enthält.

## Claims

**1.** A process for oligomerisation of isocyanates comprising a step of continuous transformation of an isocyanate monomer into polyisocyanate containing carbamate and/or allophanate groups in the presence of a catalyst, wherein the transformation step is carried out in a plate heat exchanger reactor having a specific heat exchange coefficient greater than or equal to 200 $kW/m^3/°C$, the temperature being constant and controlled inside the reactor throughout the reaction, and maintained between 65°C and 180°C.

**2.** The process according to claim 1, **characterised in that** the catalyst is chosen among the quaternary ammonium hydroxides of the following formula (I):

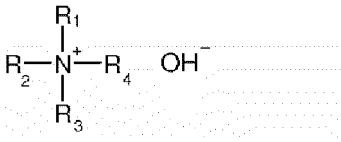

(I)

wherein:

- $R_1$ is a saturated or unsaturated alkyl group comprising 1 to 20 carbon atoms, an aryl group comprising 6 to 12 carbon atoms, a cycloalkyl group comprising 5 to 12 carbon atoms or a heterocyclic system with 5 or 6 members including a nitrogen atom, an oxygen atom or a sulfur atom,
- $R_2$, $R_3$ and $R_4$ are, independently of one another, a saturated or unsaturated alkyl group comprising 1 to 20 carbon atoms, an aryl group comprising 6 to 12 carbon atoms, said groups $R_2$, $R_3$ and $R_4$ possibly being substituted, as required, with at least one hydroxyl group or a thiol group.

3. The process according to claim 1 or 2, **characterised in that** the transformation step is carried out during a residence time in the reactor of less than 1 hour.

4. The process according to any of the claims 1 to 3, **characterised in that** the isocyanate monomer and the catalyst are added to the reactor simultaneously.

5. The process according to any of the claims 1 to 4, **characterised in that** the isocyanate monomer is chosen from the group consisting of the aliphatic, cycloaliphatic and aromatic isocyanates.

6. The process according to any of the claims 1 to 5, **characterised in that** the isocyanate monomer is chosen from the group consisting of 1,6-hexamethylene diisocyanate, 1,12-dodecane diisocyanate, cyclobutane-1,3-diisocyanate, cyclohexane-1,3 and/or 1,4-diisocyanate, 1-isocyanato-3,3,5-trimethyl-5-isocyanatomethyl cyclohexane, isocyanatomethyloctylene-diisocyanates, dicyclohexylmethane 4,4'-diisocyanate, toluene diisocyanate, methylene bisphenylisocyanate, 2,2,4-trimethyl-1,6-hexamethylene diisocyanate, 2,4- and/or 2,6-hexahydrotoluylene diisocyanate, 2,4- and/or 2,6-toluene diisocyanate, hexahydro-1,3 and/or 1,4-phenylene diisocyanate, 2-methylpentamethylene diisocyanate, 1,3- and/or 1,4-phenylene diisocyanate, tetramethyl xylylene diisocyanates, triphenylmethane-4,4',4''-triisocyanate, lysine diisocyanate as well as the esters of lysine di- or tri-isocyanate, diphenylmethane-2,4' and/or 4,4'-diisocyanate, perhydro 2,4' and/or 4,4'-diphenylmethane diisocyanate, and generally the amine aromatic precursors or the perhydrogenated carbamates, bis-isocyanatomethyl-cyclohexanes, in particular 1,3-BIC and 1,4-BIC, bis-isocyanatomethyl-norbornanes and MDI or TDI oligomers.

7. The process according to any of the claims 1 to 6, **characterised in that** the catalyst is chosen from the group consisting of tetrabutylammonium hydroxide and N,N,N-trimethyl-N-(2-hydroxyethyl)ammonium hydroxide.

8. The process according to any of the claims 1 to 7, **characterised in that** the amount of catalyst used is 0.01 % to 0.5% by weight, relative to the weight of the monomer.

9. The process according to any of the claims 1 to 8, for preparing hexamethylene diisocyanate trimer comprising a step of transforming 1,6-hexamethylene diisocyanate in the presence of N,N,N-trimethyl-N-(2-hydroxyethyl)ammonium.

10. The process according to any of the claims 1 to 8, for preparing hexamethylene diisocyanate trimer comprising a step of transforming 1,6-hexamethylene diisocyanate in the presence of tetrabutylammonium hydroxide.

11. The process according to any of the claims 1 to 10, wherein the isocyanate monomer contains more than 30 ppm of hydrolysable chlorine.

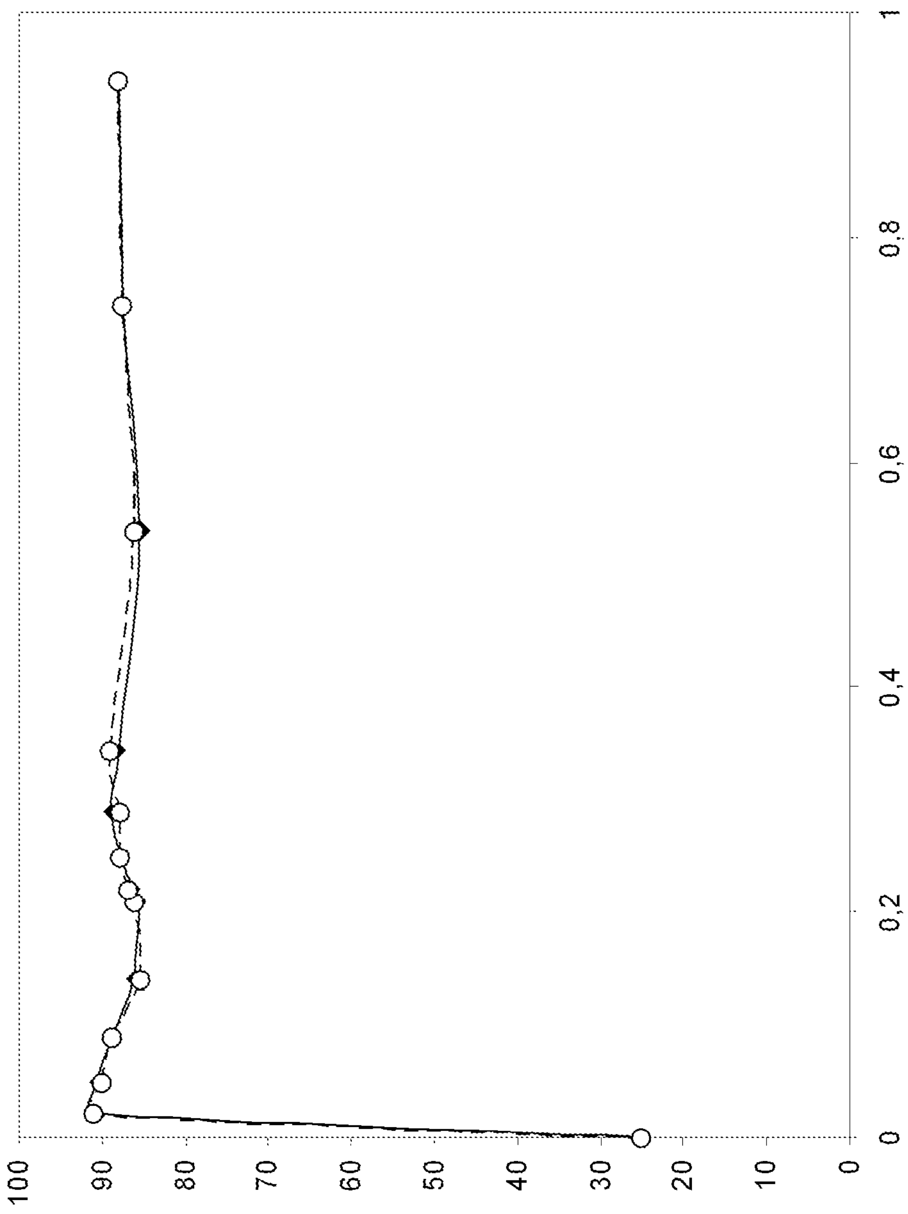


*FIG.1*

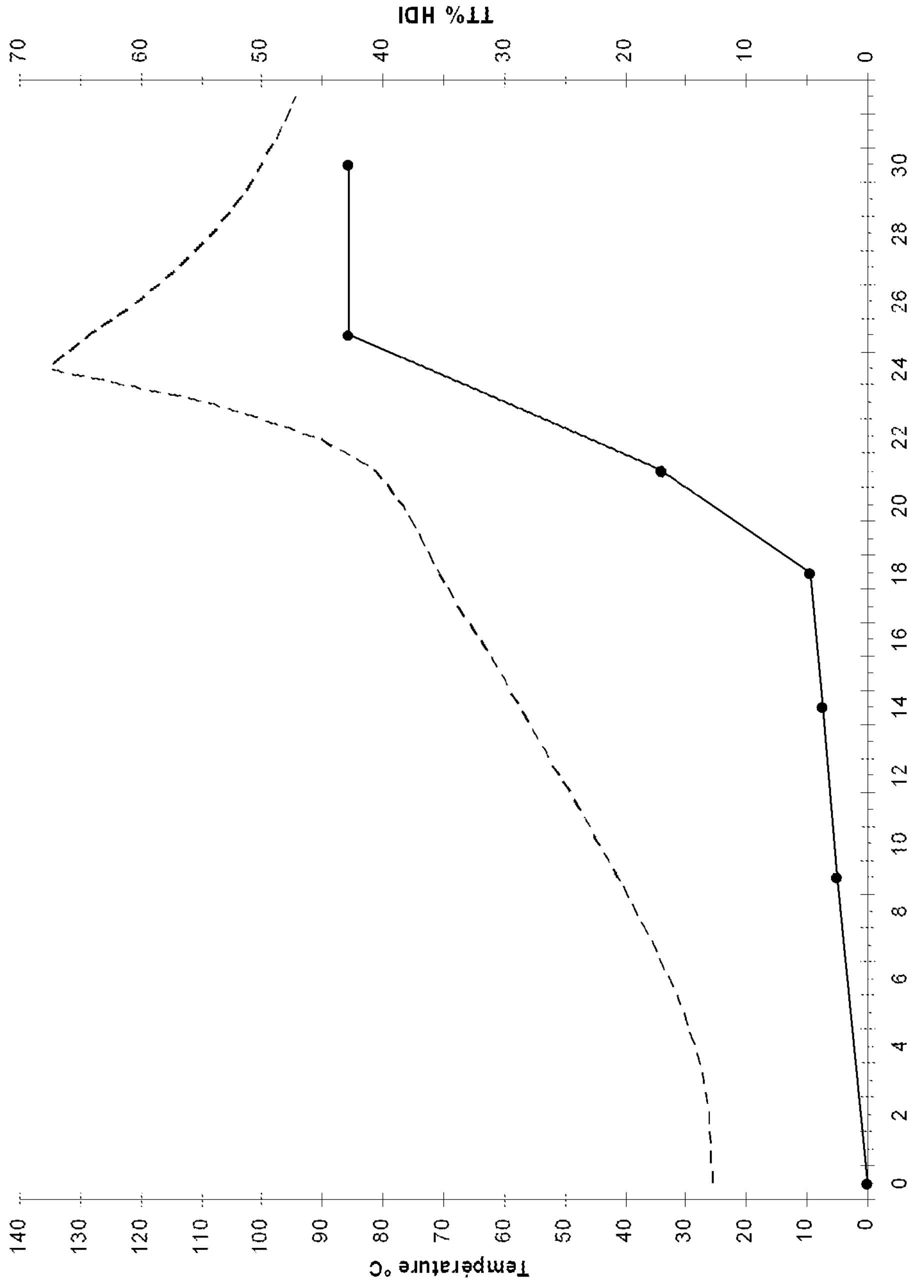
TT% HDI
70
60
50
40
30
20
10
0
Température °C
140
130
120
110
100
90
80
70
60
50
40
30
20
10
0
0
2
4
6
8
10
12
14
16
18
20
22
24
26
28
30

FIG.2

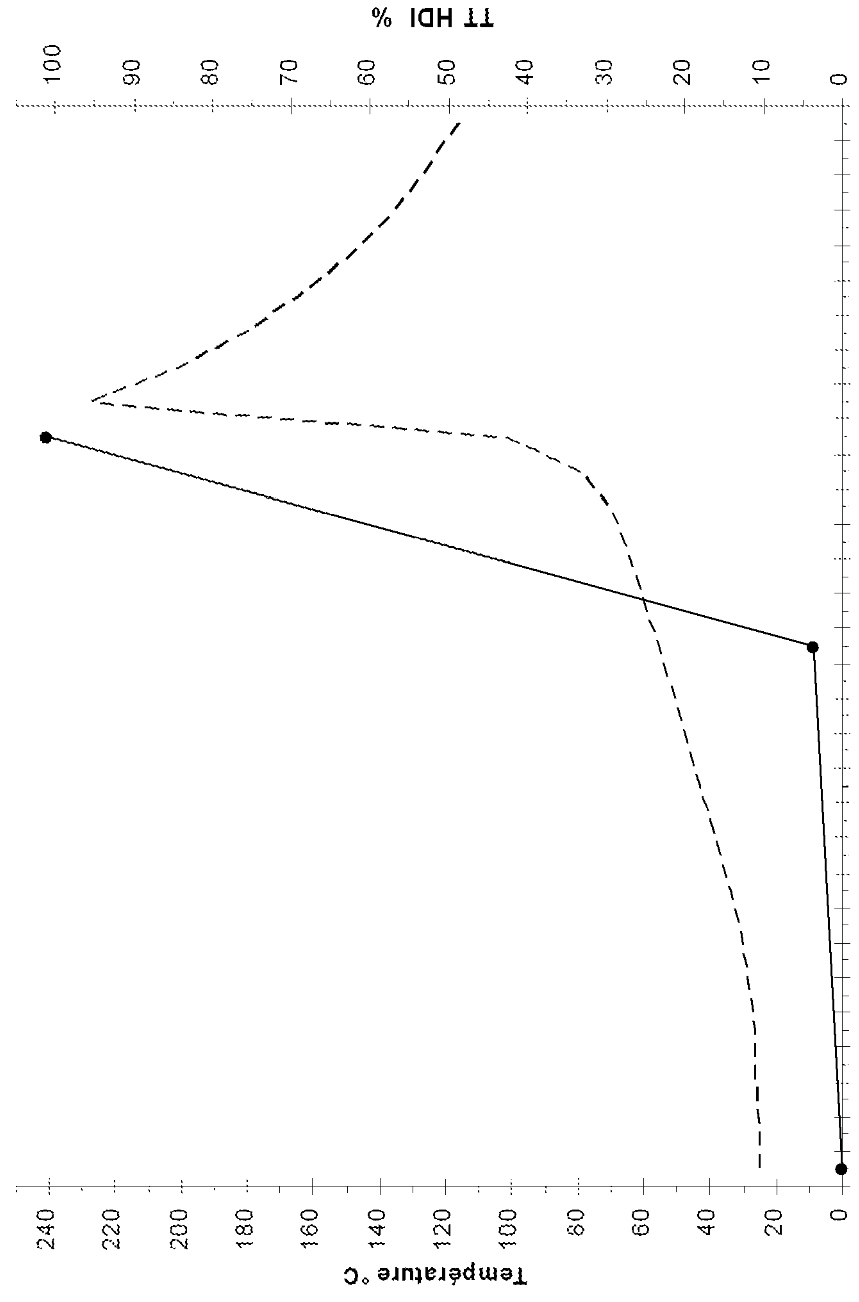
TT HDI %
100
90
80
70
60
50
40
30
20
10
0
Température °C
240
220
200
180
160
140
120
100
80
60
40
20
0
0
2
4
6
8
10
12
14
16
18
20
22
24
26
28
30

***FIG.3***

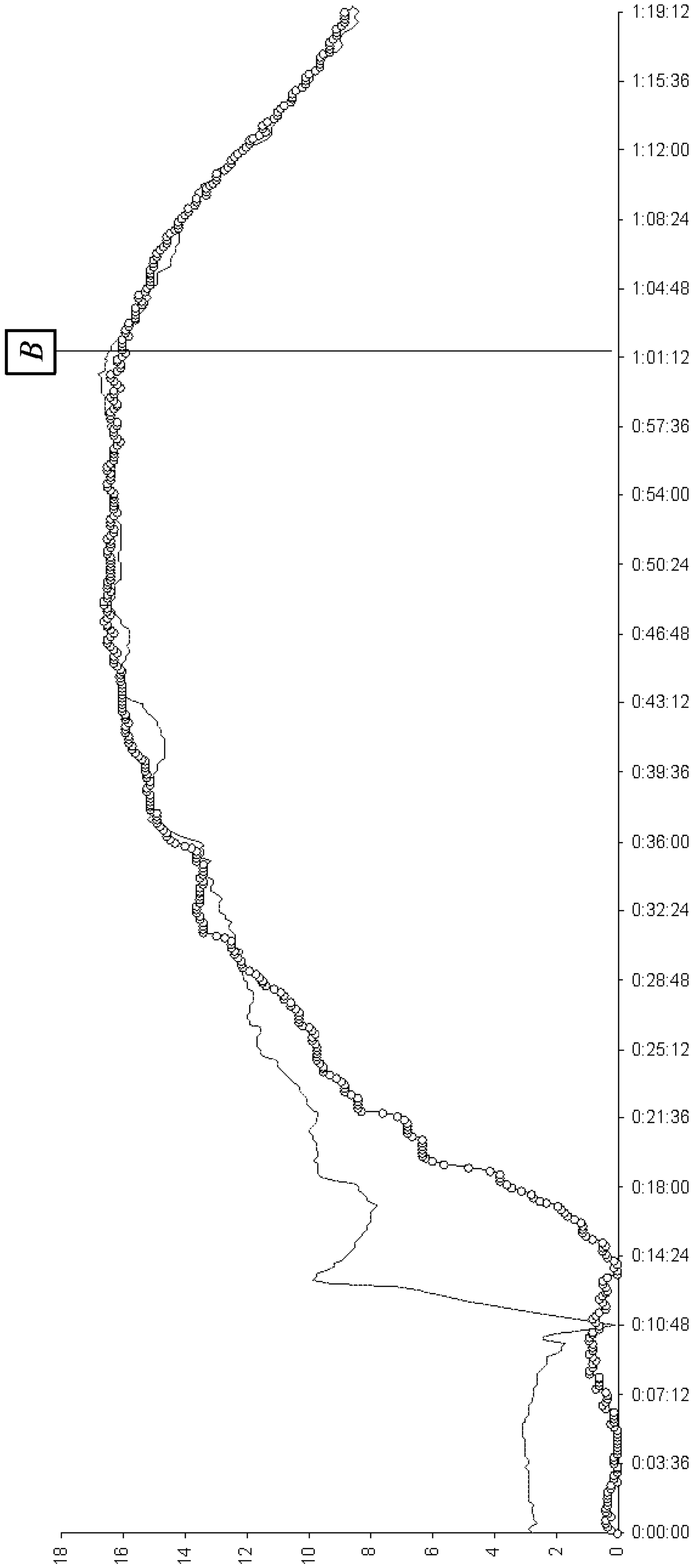
B
0:00:00
0:03:36
0:07:12
0:10:48
0:14:24
0:18:00
0:21:36
0:25:12
0:28:48
0:32:24
0:36:00
0:39:36
0:43:12
0:46:48
0:50:24
0:54:00
0:57:36
1:01:12
1:04:48
1:08:24
1:12:00
1:15:36
1:19:12
0
2
4
6
8
10
12
14
16
18

***FIG.4***

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 0003765 A
- DE 102004060131
- EP 0927731 A
- WO 0253614 A
- WO 0149766 A
- FR 2823995
- US 20040109798 A
- FR 2880967
- WO 0285511 A
- US 20060159600 A
- EP 1562699 A
- WO 0237047 A
- WO 0258840 A